# EUROPEAN PATENT SPECIFICATION

(11) **EP 3 127 516 B1**
(45) Date of publication and mention of the grant of the patent: **31.01.2018**
(21) Application number: 16001698.6
(22) Date of filing: 02.08.2016
(51) Int. Cl.: A61F 5/56, A61C 7/36

(54) **FUNCTIONAL ORTHODONTIC MOVABLE DEVICE**
FUNKTIONALE ORTHODONTISCHE BEWEGLICHE VORRICHTUNG
DISPOSITIF MOBILE ORTHODONTIQUE FONCTIONNEL

(30) Priority: 04.08.2015 IT UB20152835
(43) Date of publication of application: 08.02.2017
(73) Proprietor: Loreni, Arturo, 10151 Torino (IT)
(72) Inventor: Loreni, Arturo, 10151 Torino (IT)
(74) Representative: Aprà, Mario

(56) References cited:
- EP-A1- 0 182 387
- WO-A1-2013/061262
- DE-A1-102011 051 443
- US-A1- 2007 037 112

## Description

The present invention relates to a functional orthodontic movable device.

In particular, the present invention relates to a functional orthodontic movable device, for correcting postural defects and malocclusions, such as overbite or the like, to be worn preferably during the night.

The operation of similar functional orthodontic movable devices is essentially based on the unconscious swallowing of the person, which occurs around two thousand times in a period of twenty-four hours, in order to achieve the desired functional result. There are currently known, in the state of the art, different types of functional orthodontic movable devices.

For example, there is known a functional orthodontic movable device comprising a front bite splint resting on the occlusal surface of the upper incisors, exploiting a specific guiding plane, and a shank that is inserted into the palatal button.

It is also provided with a vestibular arch with lugs at the level of the canines and side shields made of resin (known to those skilled in the art), and with metal bilateral rear occlusal tabs, which give balance.

Said prior art device is also suitable to include a plurality of small springs to vestibularize the front elements, i.e. expansion springs.

Further embodiments of said known functional orthodontic movable device can also comprise, in particular in order to achieve an improved effect for the purposes of treating malocclusions (and, in particular, of overbite), a second front bite splint, superimposed on said first front bite splint at a distance of around 3 mm, said bite splints being joined by means of a rear hinge, in order to limit the level of overall elasticity of the device.

In this way, again exploiting the involuntary swallowing of the person, it is possible to achieve an appreciable result in the treatment of malocclusions and, in particular, of overbite.

There is also known a functional orthodontic movable device comprising two symmetrical lateral pairs of metal bite splints mutually superimposed at a slight distance, in each of which a respective bite splint rests on the occlusal surface of corresponding upper molars and premolars, and also including a vestibular arch with lugs at the level of the canines and side shields made of resin. The considerations expressed above for the device comprising a pair of front metal bite splints, mutually superimposed at a slight distance, are also valid for this latter device. Document WO2013061262 discloses an orthodontic device according to the preamble of claim 1. However, as better specified in the text below, said known functional orthodontic movable devices have some problems. Firstly, the structure of said known devices does not allow sufficient compliance with any postural and/or speech therapy requirements, particularly due to the lack of space for movement of the tongue muscle when the device is worn by the person.

Moreover, due to the presence of the shank of the double bite splint in the area of the palatal rugae, said known devices do not allow the lingual spot to remain free and the tip of the tongue to be disposed in the upper back-papillary area, in which there are at least five points known as postural exteroceptors where it is essential for the tip of the tongue to be positioned.

The present invention, taking account of the above and starting from the notion of the aforesaid problems, intends to provide a solution thereto.

An object of the present invention is to provide a functional orthodontic movable device that allows sufficient compliance with postural and/or speech therapy requirements, in particular providing a space for movement of the tongue muscle when the device is worn by the person.

Another object of the present invention is to provide a functional orthodontic movable device that does not require the presence of the shank of the double bite splint in the area of the palatal rugae.

Yet another object of the present invention is to provide a functional orthodontic movable device, which allows the lingual spot to remain free and the tip of the tongue to be disposed in the upper back-papillary area, in which there are at least five points known as postural exteroceptors where it is essential for the tip of the tongue to be positioned. Moreover, it is an object of the present invention to provide a functional orthodontic movable device which has an essentially simple structure that is easy to use and maintain, and which can be produced in a relatively simple way and with essentially limited costs.

In view of these objects, the present invention provides a functional orthodontic movable device, the essential characteristic of which forms the subject matter of claim 1. Further advantageous characteristics are described in the dependent claims.

The aforesaid claims are intended as fully incorporated herein.

In particular, according to the present invention, said functional orthodontic movable device, comprising a pair of front metal bite splints, mutually superimposed at a slight distance, one of which rests on the occlusal surface of the upper incisors, respectively two lateral symmetrical pairs of metal bite splints mutually superimposed at a slight distance, in each of which a respective bite splint rests on the occlusal surface of corresponding upper molars and premolars, and also including a vestibular arch with lugs at the level of the canines, side shields made of resin and, in combination with said pair of front bite splints, metal bilateral rear occlusal tabs, is characterized in that in each pair of said superimposed metal bite splints, a first and a second bite splint are movably connected to each other by means of a plurality of stiff filiform guiding supports, fixed with respect to one of said two bite splints, guiding the occlusal plane, the other of said bite splints being limitedly mobile in relation to said same guiding supports, substantially in parallel arrangement to said occlusal plane, and in that between said first and said second bite splints there are provided elastic means for countering the mutual closure of said first and second bite splints.

The present invention will be more apparent from the detailed description below, with reference to the accompanying drawing, illustrating an example of embodiment of the functionalizing orthodontic device according to the invention, wherein:
- Fig. 1 is a bottom plan view of the functionalizing orthodontic device according to a first example of embodiment of the present invention, in the direction of the arrow I of Fig. 3, and in which there are illustrated a pair of front metal bite splints, mutually superimposed at a slight distance;
- Fig. 2 is a top plan view in the direction of the arrow II of Fig. 3;
- Fig. 3 is a side elevation view in the direction of the arrow III of Fig. 2, illustrating two bite splints mutually spaced apart;
- Fig. 4 is a view similar to that of Fig. 3, but in which the two bite splints are positioned close to each other;
- Fig. 5 is a sectional view along the line V-V of Fig. 2;
- Fig. 6 is a perspective top half view of the device of Fig. 1;
- Fig. 7 is a front elevation view of a bite splint of the device according to the present embodiment of the invention, with related guiding supports;
- Fig. 8 is a front elevation view of the two bite splints of the device according to the present embodiment of the invention, mutually assembled and spaced apart by means of interposed elastic means;
- Fig. 9 is a view similar to that of Fig. 8, but in which said elastic means are compressed and said bite splints are positioned close to each other;
- Fig. 10 is a detailed view on a larger scale of the detail X of Fig. 8, in which there are illustrated in section countering elastic means, in uncompressed state, and in which the respective guiding supports are omitted for clarity of illustration;
- Fig. 11 is a detailed view on a larger scale of the detail XI of Fig. 9, and similar to Fig. 10, ma in which said countering elastic means are illustrated in compressed state;
- Fig. 12 is a bottom plan view of the functionalizing orthodontic device according to a second example of embodiment of the present invention, in the direction of the arrow XII of Fig. 14, in which there are illustrated two lateral symmetrical pairs of metal bite splints mutually superimposed at a slight distance;
- Fig. 13 is a top plan view in the direction of the arrow XIII of Fig. 14;
- Fig. 14 is a side elevation view of the device of Fig. 12, in which said metal bite splints are illustrated mutually superimposed at a slight distance;
- Fig. 15 is a view similar to that of Fig. 14, but in which said bite splints are positioned close to each other;
- Fig. 16 is a schematic cross-sectional view of a lateral pair of superimposed metal bite splints, in which said metal bite splints are illustrated mutually superimposed at a slight distance;
- Fig. 17 is a view similar to that of Fig. 16, but in which said bite splints are positioned close to each other;
- Fig. 18 is a front elevation view of a single bite splint of the device according to the present embodiment of the invention, with related guiding supports;
- Fig. 19 is a detailed view on a larger scale of the detail XIX of Fig. 16, in which there are illustrated in section countering elastic means, in uncompressed state, and in which respective guiding supports are omitted for clarity of illustration;
- Fig. 20 is a detailed view on a larger scale of the detail XX of Fig. 17, in a view similar to Fig. 19, but in which said countering elastic means are illustrated in compressed state;
- Fig. 21 is a sectional view on a larger scale, along the line XXI-XXI of Fig. 13, in which there are illustrated tongue shield means provided on one of the bite splints of each pair, to protect the tongue from possible pinching during compression.

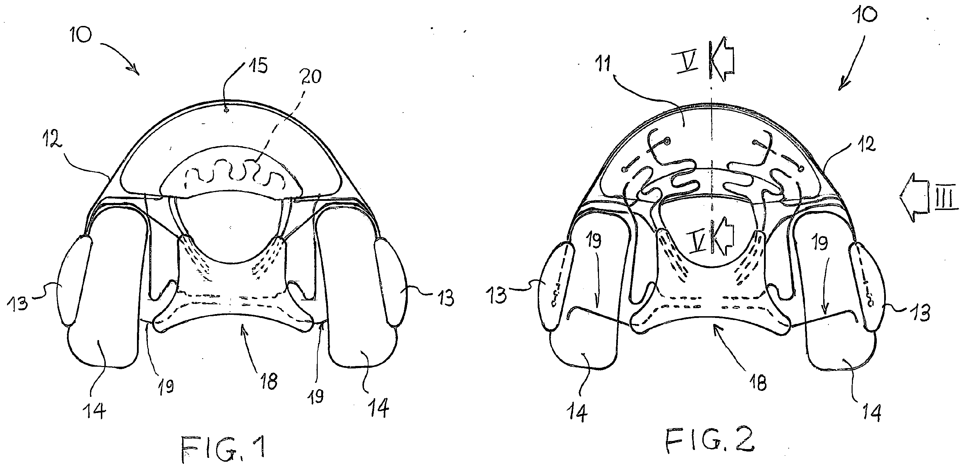

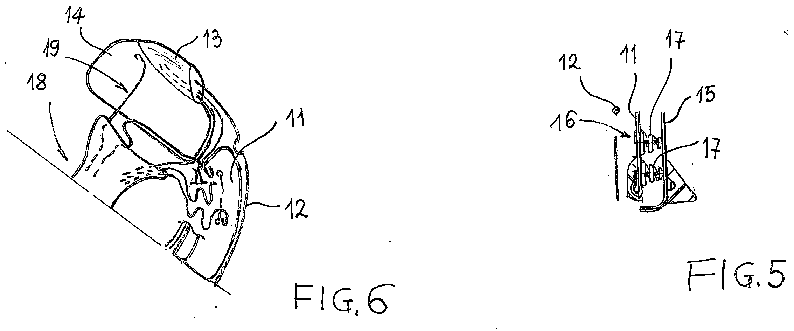

### First embodiment of the invention (Figs. 1-11)

In the present embodiment, the functional device is indicated, as a whole, with 10.

Said device 10 comprises, according to the prior art, a first front metal bite splint 11, which rests on the occlusal surface of the upper incisors, including a vestibular arch 12 with lugs at the level of the canines, side shields 13 made of resin and metal bilateral rear occlusal tabs 14, and a second bite splint 15 superimposed on said first bite splint 11 at a slight distance.

According to the present embodiment of the invention, said first 11 and said second 15 bite splints are movably connected to each other by means of a plurality of stiff filiform guiding supports 16, fixed with respect to said second bite splint 15, guiding the occlusal plane, the other of said bite splints 11 being limitedly mobile in relation to said same guiding supports 16, substantially in parallel arrangement to said occlusal plane.

Moreover, between said first 11 and said second 15 bite splints, there are provided elastic means 17 for countering the mutual closure of said first 11 and second 15 bite splints.

In this way, when the device 10 is worn by a person, said second bite splint 15 is arranged elastically floating within the limits of a stroke allowed by said guiding supports 16, with respect to said first bite splint 11, parallel to said occlusal plane, so that the lingual spot remains free and the tip of the tongue can be disposed in the upper back-papillary Advantageously, said elastic means 17 are arranged at said guiding supports 16, which pass through corresponding holes provided in said first bite splints 11 and, when said elastic means 17 are elastically compressed, said holes allow the partial escape of the same guiding supports 16 with respect to said first bite splint 11, to a back-papillary area of the oral cavity, without teeth and mucosa.

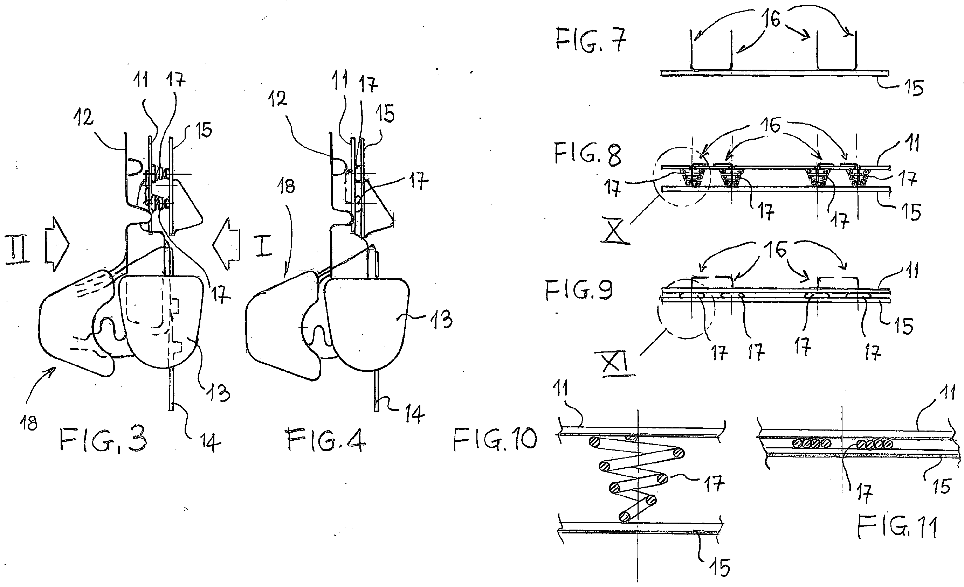

In the embodiment illustrated, said filiform guiding supports 16 have a free end thereof folded substantially to 90 degrees, extending beyond said respective holes, and resting against said first bite splint 11 (Figs. 2 and 8).

More in particular, in the present example of embodiment of the invention, said filiform guiding supports 16 are coupled and U-shaped, with vertical branches extending through corresponding holes of said first bite splint 11 and have a free end thereof folded substantially to 90 degrees, and resting against said first bite splint 11, while the intermediate portion of each pair of said supports 16 is fixed to said second bite splint 15 (Fig. 7).

In the present embodiment, said elastic means 17 advantageously consist of helical springs.

In particular, the axis of each of said helical springs 17 is substantially contained in a corresponding essentially vertical filiform element of a respective guiding support 16. As can be seen in Fig. 11, each of said helical springs 17, in fully compressed condition, is arranged as a flat packet spiral.

Advantageously, said device 10 includes a palatal button 18 essentially arch-shaped between said metal bilateral rear occlusal tabs 14, so that the lingual spot remains free (Figs. 1 to 4).

The device 10 according to the invention can also include two fixed stiff metal wires 19, having free ends respectively interposed between the sixth teeth of the mesial area and the fifth teeth of the distal area of the upper jaw bilaterally (Fig. 2).

In particular, said metal wires 19 have the other end thereof fixed with respect to said bridge 18.

Moreover, advantageously, said second bite splint 15 includes a metal wire 20 clamped in the middle of the same bite splint (Fig. 1). In particular, said metal wire 20 is embedded in resin, fixed to said second bite splint 15 (Fig. 1).

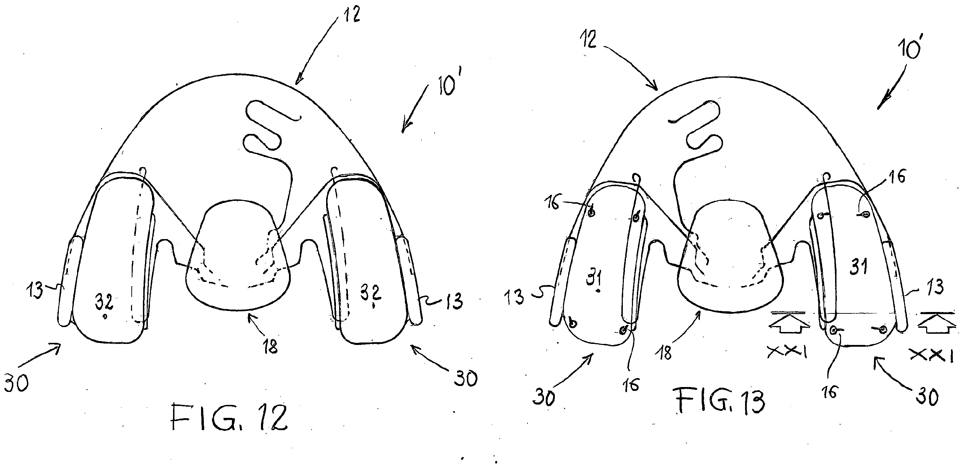

### Second embodiment of the invention (figure 12-21)

In the present embodiment, the functional device is indicated, as a whole, with 10'. In said embodiment, similar parts to those described with reference to the first embodiment are indicated with the same reference numerals.

Said device 10' includes, according to the prior art, two lateral symmetrical pairs 30 of metal bite splints 31 and 32, mutually superimposed at a slight distance, in each of which a respective bite splint 31 rests on the occlusal surface of corresponding upper molars and premolars. Said device 10' also includes a vestibular arch 12 with lugs at the level of the canines, and side shields 13 made of resin.

According to the present embodiment of the invention, in each lateral pair 30, the first 31 and the second 32 bite splints are movably connected to each other by means of a plurality of stiff filiform guiding supports 16, fixed with respect to said second bite splint 32, guiding the occlusal plane, the other of said bite splints 31 being limitedly mobile in relation to said same guiding supports 16, substantially in parallel arrangement to said occlusal plane.

Moreover, between said first 31 and said second 32 bite splints, there are provided elastic means 17 for countering the mutual closure of said first 31 and second 32 bite splints.

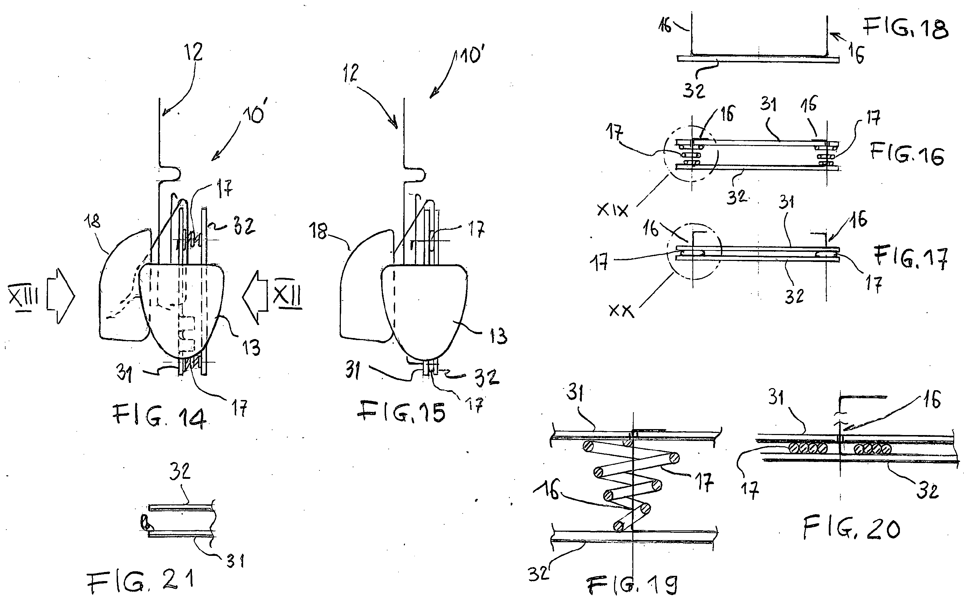

In this way, when the device 10' is worn by a person, said second bite splint 32 is arranged elastically floating within the limits of a stroke allowed by said guiding supports 16, with respect to said first bite splint 31, parallel to said occlusal plane, so that the lingual spot remains free and the tip of the tongue can be disposed in the upper back-papillary Also in the present embodiment, advantageously, said elastic means 17 are arranged at said guiding supports 16, which pass through corresponding holes provided in said first bite splint 31 and, when said elastic means 17 are elastically compressed, said holes allow the partial escape of the same guiding supports 16 with respect to said first bite splint 31, to a back-papillary area of the oral cavity, without teeth and mucosa (Fig. 17).

In the embodiment illustrated, said filiform guiding supports 16 have a free end thereof folded substantially to 90 degrees, extending beyond said respective holes, and resting against said first bite splint 31 (Figs. 13, 16 and 19).

More in particular, in the present second example of embodiment of the invention, said filiform guiding supports 16 are coupled and U-shaped (Fig. 18), with vertical branches extending through corresponding holes of said first bite splint 31 and having a free end thereof folded substantially to 90 degrees, and resting against said first bite splint 31, while the intermediate portion of each pair of said supports 16 is fixed to said second bite splint 32 (Figs. 18 and 19).

In the present embodiment, said elastic means 17 advantageously consist of helical springs.

In particular, the axis of each of said helical springs 17 is substantially contained in a corresponding essentially vertical filiform element of a respective guiding support 16. As can be seen in Fig. 20, each of said helical springs 17, in fully compressed condition, is arranged as a flat packet spiral.

Advantageously, said device 10' includes a palatal button 18 essentially arch-shaped between said two lateral symmetrical pairs 30 of metal bite splints 31, 32 superimposed, so that the lingual spot remains free (Figs. 12 to 15).

As is apparent from the considerations above, said functional orthodontic movable device according to the invention allows sufficient compliance with postural and/or speech therapy requirements, in particular providing space for movement of the tongue muscle when the device is worn by the person.

Moreover, said device as indicated does not require the presence of the shank of the double bite splint in the palatal rugae area.

In addition, said device as specified allows the lingual spot to remain free and the tip of the tongue to be disposed in the upper back-papillary area, in which there are at least five points known as postural exteroceptors where it is essential for the tip of the tongue to be positioned.

Moreover, said device as mentioned has an essentially simple structure that is easy to use and maintain, and which can be produced in a relatively simple way and with essentially limited costs. As is apparent from the above, the present invention allows the objects set forth in the introduction to be achieved in a simple and advantageous manner.

## Claims

1. Functional orthodontic movable device (10; 10') comprising a pair of front metal bite splints, mutually superimposed at a slight distance, one of which rests on the occlusal surface of the upper incisors, respectively two lateral symmetrical pairs (30) of metal bite splints (31, 32), mutually superimposed at a slight distance, in each of which a respective bite splint (31) rests on the occlusal surface of corresponding upper molars and premolars, and also including a vestibular arch (12) with lugs at the level of the canines, side shields (13) made of resin and, in combination with said pair of front bite splints, metal bilateral rear occlusal tabs (14) ,
**characterized in that**
in each pair of said superimposed metal bite splints, a first (11; 31) and a second (15; 32) bite splint are movably connected to each other by means of a plurality of stiff filiform guiding supports (16), fixed with respect to one of said two bite splints (11, 15; 31, 32), guiding the occlusal plane, the other of said bite splints (11, 15; 31, 32) being limitedly mobile in relation to said same guiding supports (16), substantially in parallel arrangement to said occlusal plane,
**and in that** between said first (11; 31) and said second (15; 32) bite splints there are provided elastic means (17) for countering the mutual closure of said first (11; 31) and second (15; 32) bite splints,
so that, when worn by a person, said second bite splint (15; 32) is arranged elastically floating within the limits of a stroke allowed by said guiding supports (16), with respect to said first bite splint (11; 31), parallel to said occlusal plane, so that the lingual spot remains free and the tip of the tongue can be disposed in the upper back-papillary area.

2. Device (10; 10') according to claim 1, **characterized in that** said elastic means (17) are arranged at said guiding supports (16), which pass through corresponding holes provided in one of said bite splints (11; 31) and, when said elastic means (17) are elastically compressed, said holes allow the partial escape of the same guiding supports (16) with respect to said bite splint (11; 31) to a back-papillary area of the oral cavity, without teeth and mucosa.

3. Device (10; 10') according to claim 2, **characterized in that** said filiform guiding supports (16) have a free end thereof folded substantially to 90 degrees, extending beyond said respective holes, and resting against one of said bite splints (11; 31).

4. Device (10; 10') according to claim 3, **characterized in that** said filiform guiding supports (16) are coupled and U-shaped, with vertical branches extending through corresponding holes of one (11; 31) of said bite splints and having a free end thereof folded substantially to 90 degrees, and resting against one (11; 31) of said bite splints, the intermediate portion of each pair of said supports (16) being fixed to the other (15; 32) of said bite splints.

5. Device (10; 10') according to one or more of the preceding claims, **characterized in that** said elastic means (17) consist of helical springs.

6. Device (10; 10') according to claim 5, **characterized in that** the axis of each of said helical springs (17) is substantially contained in a corresponding essentially vertical filiform element of a respective guiding support (16) .

7. Device (10; 10') according to claims 5 and/or 6, **characterized in that** each of said helical springs (17), in fully compressed condition, is arranged as a flat packet spiral.

8. Device (10; 10') according to one or more of the preceding claims, **characterized in that** said device (10) includes a palatal button (18) essentially arch-shaped between said metal bilateral rear occlusal tabs (14), so that the lingual spot remains free.

9. Device (10) according to one or more of the preceding claims, **characterized in that** it includes two fixed stiff wires (19) having free ends respectively interposed between the sixth teeth of the mesial area and the fifth teeth of the distal area of the upper jaw bilaterally.

10. Device (10) according to one or more of the preceding claims, **characterized in that** said second bite splint (15) includes a metal wire (20) clamped in the middle of the same bite splint.

## Patentansprüche

1. Ein bewegliches funktionskieferothopädisches Gerät (10; 10'), umfassend ein vorderes Paar Metall-Aufbissschienen, die einander mit geringem Abstand überlagern, von denen eine auf der Kaufläche der oberen Schneidezähne ruht, beziehungsweise zwei symmetrische Seitenpaare (30) von Metall-Aufbissschienen (31, 32), die einander mit geringem Abstand überlagern, von denen jeweils eine diesbezügliche Aufbissschiene (31) auf der Kaufläche der entsprechenden oberen Molarzähne und Prämolarzähne ruht, und umfassend ferner einen Vestibularbogen (12) mit Einbuchtungen in Höhe der Eckzähne, Seitenschilder (13) aus Kunststoff und, kombiniert mit dem besagten vorderen Aufbissschienen-Paar, hintere okklusale bilaterale Metall-Tabs (14),
**gekennzeichnet dadurch, dass**
bei jedem Paar der besagten überlagerten Metall-Aufbissschienen eine erste (11; 31) und eine zweite (15; 32) Aufbissschiene miteinander beweglich verbunden sind durch eine Vielzahl von starren, fadenförmigen Führungshalterungen (16), befestigt in Bezug auf eine der besagten zwei Aufbissschienen (11, 15; 31, 32) zur Führung der Okklusalebene, die andere der besagten Aufbissschienen (11, 15; 31, 32), in Bezug auf die besagten selbigen Führungshalterungen (16) begrenzt beweglich, ist im Wesentlichen in Bezug auf die besagte Okklusalebene parallel ausgerichtet,
**und dadurch, dass** zwischen der besagten ersten (11; 31) und der besagten zweiten (15; 32) Aufbissschiene elastische Mittel (17) zum Entgegenwirken des gegenseitigen Verschlusses der besagten ersten (11; 31) und zweiten (15; 32) Aufbissschiene vorgesehen sind,
so dass, wenn von einer Person getragen, die besagte zweite Aufbissschiene (15; 32) elastisch fluktuierend angeordnet ist innerhalb der Grenzen eines Hubs, der von den besagten Führungshalterungen (16) in Bezug auf die besagte erste Aufbissschiene (11; 31), parallel zu der besagten Okklusalebene, erlaubt wird, so dass der Zungen-Spot frei bleibt und sich die Zungenspitze in den oberen hinteren papillaren Bereich stellen kann.

2. Gerät (10; 10') gemäß Anspruch 1, **gekennzeichnet dadurch, dass** besagte elastische Mittel (17) in Übereinstimmung mit den besagten Führungshalterungen (16) angeordnet sind, die entsprechende Löcher durchlaufen, die in einer der besagten Aufbissschienen (11; 31) vorgesehen sind, und, wenn besagte elastische Mittel (17) elastisch komprimiert werden, erlauben besagte Löcher das teilweise Austreten der besagten selbigen Führungshalterungen (16) in Bezug auf die besagte Aufbissschiene (11; 31) in einen hinteren papillaren Bereich der Mundhöhle, ohne Zähne und Schleimhaut.

3. Gerät (10; 10') gemäß Anspruch 2, **gekennzeichnet dadurch, dass** besagte fadenförmige Führungshalterungen (16) ein freies Ende aufweisen, das im Wesentlichen in einem 90-Grad-Winkel gefaltet ist, und sich jenseits der besagten diesbezüglichen Löcher erstreckt und gegen eine der besagten Aufbissschienen (11; 31) ruht.

4. Gerät (10; 10') gemäß Anspruch 3, **gekennzeichnet dadurch, dass** besagte fadenförmige Führungshalterungen (16) gekoppelt und U-förmig geformt sind, deren vertikale Äste sich durch entsprechende Löcher von einer der (11; 31) besagten Aufbissschienen erstrecken und ein freies Ende aufweisen, das im Wesentlichen in einem 90-Grad-Winkel gefaltet ist, und gegen eine (11; 31) der besagten Aufbissschienen ruht, wobei der mittlere Abschnitt eines jeden Paars der besagten Halterungen (16) an der anderen (15; 32) der besagten Aufbissschienen befestigt ist.

5. Gerät (10; 10') gemäß einer oder mehrerer der vorhergehenden Ansprüche, **gekennzeichnet dadurch, dass** besagte elastische Mittel (17) aus Schraubenfedern bestehen.

6. Gerät (10; 10') gemäß Anspruch 5, **gekennzeichnet dadurch, dass** die Achse einer jeden der besagten Schraubenfedern (17) im Wesentlichen in einem im Wesentlichen vertikalen entsprechenden fadenförmigen Element einer diesbezüglichen Führungshalterung (16) enthalten ist.

7. Gerät (10; 10') gemäß den Ansprüchen 5 und/oder 6, **gekennzeichnet dadurch, dass** jede der beiden Schraubenfedern (17), in vollständig komprimiertem Zustand, als paketförmige flache Spirale angeordnet ist.

8. Gerät (10; 10') gemäß einer oder mehrerer der vorhergehenden Ansprüche, **gekennzeichnet dadurch, dass** besagtes Gerät (10) einen palatalen Knopf (18) umfasst, der im Wesentlichen als Bogen zwischen den besagten hinteren okklusalen bilateralen Metall-Tabs geformt ist, so dass der Zungen-Spot frei bleibt.

9. Gerät (10) gemäß einer oder mehrerer der vorhergehenden Ansprüche, **gekennzeichnet dadurch, dass** es zwei feste starre Metalldrähte (19) umfasst, die freie Enden aufweisen, die jeweils zwischen den sechsten Zähnen des mesialen Bereichs und den fünften Zähnen des distalen Bereichs des Oberkiefers auf beiden Seiten eingefügt sind.

10. Gerät (10) gemäß einer oder mehrerer der vorhergehenden Ansprüche, **gekennzeichnet dadurch, dass** besagte zweite Aufbissschiene (15) einen Metalldraht (20) umfasst, der in der Mitte der selbigen Aufbissschiene eingespannt ist.

## Revendications

1. Dispositif (10 ; 10') fonctionnel mobile de type orthodontique, comprenant une paire antérieure de plaques occlusales métalliques superposées entre elles avec une légère distance, dont une repose sur la surface occlusale des incisives supérieures, respectivement deux paires (30) latérales symétriques de plaques occlusales métalliques (31, 32) superposées entre elles avec une légère distance, dans chacune desquelles une plaque occlusale respective (31) repose sur la surface occlusale de molaires et prémolaires supérieures correspondantes, et incluant également un arc vestibulaire (12) avec des anses au niveau des canines, de protections latérales (13) en résine et, en combinaison avec ladite paire antérieure de plaques occlusale, des ailettes occlusales (14) métalliques bilatérales postérieures,
**caractérisé par la fait que**
dans chaque paire desdites plaques occlusales métalliques superposées, une première (11 ; 31) et une seconde (15 ; 32) plaque occlusale sont reliées entre elles de manière mobile au moyen d'une pluralité de supports de guidage (16) rigides, filiformes, fixés par rapport à l'une desdites deux plaques occlusales (11, 15 ; 31, 32), au guide du plan occlusal, l'autre desdites plaques occlusales (11, 15 ; 31, 32) ayant une mobilité limitée par rapport auxdits supports de guidage (16), substantiellement en disposition parallèle par rapport audit plan occlusal,
**et par le fait qu**'entre ladite première (11 ; 31) et ladite seconde plaque occlusale (15 ; 32), sont prévus des moyens élastiques (17) de contraste de la fermeture réciproque desdites première (11 ; 31) et seconde (15 ; 32) plaques occlusales,
de sorte que, lorsqu'il est porté par une personne, ladite seconde plaque occlusale (15 ; 32) est disposé de manière flottante et élastique, dans les limites d'une course autorisée par lesdits supports de guidage (16), par rapport à ladite première plaque occlusale (11 ; 31), parallèlement audit plan occlusal, de sorte que le spot lingual reste libre et que la pointe de la langue puisse se disposer dans la zone rétro-papillaire supérieure.

2. Dispositif (10 ; 10') selon la revendication 1, **caractérisé par la fait que** lesdits moyens élastiques (17) sont disposés en correspondance desdits supports de guidage (16), lesquels traversent des trous correspondants prévus dans l'unes desdites plaques occlusales (11 ; 31) et, quand lesdits moyens élastiques (17) sont comprimés élastiquement, lesdits trous permettent la sortie partielle desdits supports de guidage (16) par rapport à ladite plaque occlusale (11 ; 31), dans une zone rétro-papillaire de la cavité buccale, exempte de dents et de muqueuse.

3. Dispositif (10 ; 10') selon la revendication 2, **caractérisé par le fait que** lesdits supports de guidage (16) filiformes ont une de leurs extrémités libre repliée substantiellement à 90 degrés, étendue au-delà desdits trous respectifs, et appuyée contre l'une desdits plaques occlusales (11 ; 31).

4. Dispositif (10 ; 10') selon la revendication 3, **caractérisé par le fait que** lesdits supports de guidage (16) filiformes sont couplés et conformés en U, où les branches verticales s'étendent à travers des trous correspondants d'une desdites plaques occlusales (11 ; 31) et ont une de leurs extrémités libre repliée substantiellement à 90 degrés, et appuyée contre l'une desdites plaques occlusales (11 ; 31), le tronçon intermédiaire de chaque paire desdits supports (16) étant fixé à l'autre desdites plaques occlusales (15 ; 32).

5. Dispositif (10 ; 10') selon une ou plusieurs des revendications précédentes, **caractérisé par la fait que** lesdits moyens élastiques (17) sont composés par des ressorts hélicoïdaux.

6. Dispositif (10 ; 10') selon la revendication 5, **caractérisé par la fait que** l'axe de chacun desdits ressorts hélicoïdaux (17) est substantiellement contenu dans un élément filiforme correspondant essentiellement vertical d'un support de guidage respectif (16).

7. Dispositif (10 ; 10') selon les revendications 5 et/ou 6, **caractérisé par le fait que** chacun desdits ressorts hélicoïdaux (17), en condition complètement comprimé, est disposé en forme de spirale plate en paquet.

8. Dispositif (10 ; 10') selon une ou plusieurs des revendications précédentes, **caractérisé par le fait que** ledit dispositif (10) comprend un bouton palatin (18) essentiellement conformé en arc entre lesdites ailettes occlusales métalliques (14) bilatérales postérieures, de sorte que le spot lingual reste libre.

9. Dispositif (10) selon une ou plusieurs des revendications précédentes, **caractérisé par le fait qu'**il comprend deux fils métalliques (19) fixes, rigides, possèdent des extrémités libres respectivement intercalées entre les sixièmes dents de la zone mésiale et les cinquièmes dents dans la zone distale de l'arcade supérieure bilatéralement.

10. Dispositif (10) selon une ou plusieurs des revendications précédentes, **caractérisé par le fait qua** ladite seconde plaque occlusale (15) comprend un fil métallique (20), fixé au centre de la plaque occlusale.
